# EUROPÄISCHE PATENTSCHRIFT

(11) **EP 1 387 820 B1**
(45) Veröffentlichungstag und Bekanntmachung des Hinweises auf die Patenterteilung: **08.03.2006**
(21) Anmeldenummer: 02735325.9
(22) Anmeldetag: 02.05.2002
(51) Int. Cl.: C07B 63/00, C07C 57/07

(54) **VERFAHREN ZUR HERSTELLUNG EINER GEREINIGTEN SCHMELZE WENIGSTENS EINES MONOMEREN**
METHOD FOR THE PRODUCTION OF A PURIFIED MELT OF AT LEAST ONE MONOMER
PROCEDE DE PRODUCTION D'UNE MASSE FONDUE PURIFIEE D'AU MOINS UN MONOMERE

(30) Priorität: 10.05.2001 DE 10122787
(43) Veröffentlichungstag der Anmeldung: 11.02.2004
(73) Patentinhaber: BASF Aktiengesellschaft, 67056 Ludwigshafen (DE)
(72) Erfinder: HAMMON, Ulrich, 68163 Mannheim (DE); ECK, Bernd, 68519 Viernheim (DE); BAUMANN, Dieter, 67227 Frankenthal (DE); HEILEK, Jörg, 69245 Bammental (DE); MÜLLER-ENGEL, Klaus, Joachim, 76297 Stutensee (DE)
(86) Internationale Anmeldenummer: PCT/EP2002/004793
(87) Internationale Veröffentlichungsnummer: WO 2002/090299

(56) Entgegenhaltungen:
- EP-A- 0 847 978
- DE-A- 19 926 082

## Beschreibung

Vorliegende Erfindung betrifft ein Verfahren zur Herstellung einer gereinigten Schmelze wenigstens eines Monomeren, bei dem man in einer ersten Verfahrensstufe eine das wenigstens eine Monomere enthaltende Gas- oder Flüssigphase erzeugt, in einer ersten Reinigungsstufe das wenigstens eine Monomere aus der Gas- oder Flüssigphase durch Kondensation, Absorption oder Extraktion unter Erhalt einer Roh-Schmelze des wenigstens einen Monomeren abtrennt und anschließend in einer zweiten Reinigungsstufe das wenigstens eine Monomere aus der Roh-Schmelze kristallisativ abscheidet.

Der Begriff Monomere soll in dieser Schrift chemische Verbindungen umfassen, die wenigstens eine ethylenisch ungesättigte Doppelbindung aufweisen.

Aufgrund der wenigstens einen ethylenisch ungesättigten Doppelbindung bilden Monomere äußerst reaktionsfähige Verbindungen, die unter anderem zur Herstellung von Polymerisaten Verwendung finden. Typische Beispiele für Monomere sind die Acrylsäure, die Methacrylsäure und das N-Vinylpyrrolidon.

Üblicherweise werden Monomere durch chemische Synthese erzeugt. Dabei werden sie jedoch nicht unmittelbar in reiner Form befindlich sondern als Bestandteile von gasförmigen oder flüssigen Mischungen erhalten, aus denen sie abgetrennt werden müssen. Diese gasförmigen oder flüssigen Mischungen können das Ergebnis mehrerer aufeinanderfolgender Verfahrensschritte (einschließlich des eigentlichen Syntheseschrittes) sein, die hier zusammenfassend als "erste Verfahrensstufe" bezeichnet werden sollen. Ein solcher letzter Verfahrensschritt der ersten Verfahrensstufe kann z.B. eine Destillation oder Rektifikation sein, bei der das Monomer gemeinsam mit es begleitenden Verunreinigungen in der Gasphase am Kolonnenkopf (bzw. im oberen Teil der Kolonne) anfällt und durch Kondensation (erste Reinigungsstufe) der selben abgetrennt wird (vgl. z.B. die Gewinnung von N-Vinylpyrrolidon gemäß der DE-A 10026233). Der letzte Verfahrensschritt der ersten Verfahrensstufe kann jedoch auch, wie z.B. im Fall der Herstellung von Acrylsäure, eine direkte Kühlung des heißen Gasgemisches einer heterogen katalysierten Gasphasenoxidation mit einer Quenchflüssigkeit sein, wie es z.B. die DE-A 19924533 und die DE-A 10053086 beschreiben. In diesem Fall wird die Acrylsäure ebenfalls durch Kondensation (erste Reinigungsstufe), nämlich durch fraktionierte Kondensation, aus der Gasgemischphase abgetrennt. Bei einer älteren Verfahrensweise zur Herstellung von Acrylsäure wird die Acrylsäure durch Absorption (erste Reinigungsstufe) in ein Lösemittel aus der Gasgemischphase abgetrennt (vgl. z.B. DE-A 10115277).

Vielfach fällt das interessierende Monomere im letzten Verfahrensschritt der ersten Verfahrensstufe aber auch als Bestandteil einer Flüssigphase an, aus der es durch Extraktion in eine andere Flüssigkeit hinein (erste Reinigungsstufe) abgetrennt wird.

In allen beschriebenen Fällen wird so ohne Beteiligung von Feststoffen als Ergebnis der ersten Reinigungsstufe eine die interessierenden Monomere als Hauptbestandteil enthaltende Flüssigkeit erhalten, die frei von Feststoffen sein sollte.

Diese fällt dann unter den in dieser Schrift verwendeten Begriff der Roh-Schmelze des wenigstens einen Monomeren, wenn sich beim Abkühlen derselben als erster Feststoff Kristalle des wenigstens einen Monomeren abscheiden, die weniger von dem wenigstens einen Monomeren verschiedene Substanzen enthalten als die Roh-Schmelze selbst.

D.h., der hier verwendete Begriff Roh-Schmelze trifft dann nicht, wenn sich beim Abkühlen anstelle des wenigstens einen Monomeren als erster Feststoff z.B. Extraktionsmittel oder eine andere Komponente abscheidet.

In der Regel enthalten die erfindungsgemäß wesentlichen Roh-Schmelzen des wenigstens einen Monomeren geringe Mengen sogenannter Polymerisationsinhibitoren in Lösung befindlich (vgl. z.B. DE-A 19938841) zugesetzt, die eine unerwünschte radikalische Polymerisation des wenigstens einen Monomeren unter Einwirkung von Wärme und/oder Licht unterdrücken sollen.

Aus den wie vorstehend definierten Roh-Schmelzen des wenigstens einen Monomeren kann nun in an sich bekannter Weise durch Einwirkung von Kälte das wenigstens eine Monomere kristallisativ abgeschieden und so eine gereinigte Schmelze (in fester oder flüssiger Form) des wenigstens einen Monomeren hergestellt werden (vgl. z.B. DE-A 19926082, WO 00/45928, WO 94/18166, DE-A 10026407, DE-A 10039025, DE-A 10003498 und DE-A 10003497). Dabei können unterschiedlichste Kristallisationsverfahren zur Anwendung kommen. Bei den Schichtkristallisationsverfahren wird das wenigstens eine Monomere in Form zusammenhängender, fest anhaftender Schichten ausgefroren.

Die Fest/Flüssig-Trennung erfolgt durch einfaches Abfließen lassen der Restschmelze. Anschließend kann das gereinigte Kristallisat aufgeschmolzen oder in einem gewollten Lösungsmittel zur weiteren Verwendung gelöst werden.

Pinzipiell unterscheidet man zwischen "statischen" und "dynamischen" Schichtkristallisationsverfahren.

Bei den statischen Verfahren wird die zu reinigende Roh-Schmelze z.B. in Rohrbündel- oder modifizierte Plattenwärmeaustauscher eingefüllt und anschließend durch langsame Temperatursenkung auf der Sekundärseite teilweise erstarrt. Nach dem Ausfrieren wird die Restschmelze abgelassen und danach die abgeschiedene Kristallschicht als gereinigte Schmelze (Rein-Schmelze), gegebenenfalls in Stufen, abgeschmolzen. Der Wärme- und Stofftransport zu den Abscheideflächen erfolgt nur durch freie Konvektion.

Kennzeichnend für die dynamische Schichtkristallisation von Roh-Schmelzen ist eine erzwungene Konvektion der Roh-Schmelze. Diese kann durch Umpumpen der Roh-Schmelze durch volldurchströmte Rohre (z.B. DE-OS 2606364), durch Aufgabe der Roh-Schmelze als Rieselfilm (z.B. EP-A 616998) oder durch Einleiten von Inertgas in ein mit Schmelze gefülltes Rohr oder durch Pulsieren erfolgen.

Bei den Suspensionskristallisationsverfahren wird aus der Roh-Schmelze durch Kälteeinwirkung eine Kristallsuspension erzeugt, die die abgeschiedenen Kristalle in der Restschmelze suspendiert enthält. Dabei können die Feststoffkristalle unmittelbar in Suspension befindlich wachsen oder sich als Schicht auf einer gekühlten Wand abscheiden, von der sie anschließend abgekratzt und in der Restschmelze resuspendiert werden. Die Abtrennung der abgeschiedenen Kristalle von der Restschmelze kann bei einer Kristallsuspension rein mechanisch durch Abpressen, Filtrieren, Zentrifugieren und/oder in Waschkolonnen erfolgen.

Charakteristisch für alle Kristallisationsverfahren ist, daß sie Engstellen, d.h. Stellen mit engem Strömungsquerschnitt entweder für die Roh-Schmelze, die Rein-Schmelze und/oder die Restschmelze, aufweisen.

So enthalten Fallfilmkristaller z.B. üblicherweise Einbauten, die nur einen kleinen Durchflußquerschnitt frei lassen. Das Überwinden dieser Engstelle gelingt der Roh-Schmelze nur als dünner Film. Hinter der Engstelle bleibt dieser Film erhalten und fließt als Rieselfilm eine gekühlte Wandung herunter, auf der sich während des Fließprozesses Kristallisat abscheidet (vgl. z.B. EP-B 218545).

Die Trennung einer Kristallsuspension in Kristalle und Restschmelze erfolgt praktisch immer über Querschnitte, die nur für die Restschmelze nicht aber für die suspendierten Kristalle durchläßig sind (z.B. über ein zwei- oder dreidimensionales Netzwerk solcher Querschnitte beim Filtrieren oder in einer Siebzentrifuge).

Üblicherweise werden die relevanten Verfahren zur Herstellung von gereinigten Schmelzen wenigstens eines Monomeren durch kristallisative Reinigung einer Roh-Schmelze mehr oder weniger kontinuierlich (bzw. halbkontinuierlich) durchgeführt. Voraussetzung für hohe Raum-Zeit-Ausbeuten und lange Laufzeiten ist dabei, daß die beschriebenen engen Strömungsquerschnitte nicht blockiert werden.

Bei der praktischen Durchführung der relevanten Verfahren zur Herstellung gereinigter Schmelzen wenigstens eines Monomeren (insbesondere im Fall von Acrylsäure, Methacrylsäure und N-Vinylpyrrolidon) traten jedoch immer wieder in unerwarteter Weise solche Blockaden auf. Dies insbesondere dann, wenn die charakteristische Länge der Engstellen ≤ 5 mm betrug. Bemerkenswerterweise bestand das die Blockade verursachende Material nicht aus Kristallisat, da die Blockade durch Erwärmen über den Schmelzpunkt des Kristallisats hinaus im Regelfall nicht beseitigt werden konnte.

Im Unterschied dazu betrifft die EP-A 847978 die Abkühlung wässriger (Meth)acrylsäure-Lösungen, wobei sich als Nebenkomponente enthaltende Terephthalsäure abscheidet.

Die Aufgabe der vorliegenden Erfindung bestand daher darin, ein Verfahren zur Herstellung einer gereinigten Schmelze wenigstens eines Monomeren, bei dem man in einer ersten Verfahrensstufe eine das wenigstens eine Monomere enthaltende Gas- oder Flüssigphase erzeugt, in einer ersten Reinigungsstufe das wenigstens eine Monomere aus der Gas- oder Flüssigphase durch Kondensation, Absorption oder Extraktion unter Erhalt einer Roh-Schmelze des wenigstens einen Monomeren abtrennt und anschließend in einer zweiten Reinigungsstufe das wenigstens eine Monomere aus der Roh-Schmelze kristallisativ abscheidet, zur Verfügung zu stellen, bei dem solche Blockaden allenfalls noch in einem deutlich verringerten Umfang auftreten.

Demgemäß wurde ein Verfahren zur Herstellung einer gereinigten Schmelze wenigstens eines Monomeren, bei dem man in einer ersten Verfahrensstufe eine das wenigstens eine Monomere enthaltende Gas- oder Flüssigphase erzeugt, in einer ersten Reinigungsstufe das wenigstens eine Monomere aus der Gas- oder Flüssigphase durch Kondensation, Absorption oder Extraktion unter Erhalt einer Roh-Schmelze des wenigstens einen Monomeren abtrennt und anschließend in einer zweiten Reinigungsstufe das wenigstens eine Monomere aus der Roh-Schmelze kristallisativ abscheidet, gefunden, das dadurch gekennzeichnet ist, daß die Roh-Schmelze auf ihrem Weg aus der ersten Reinigungsstufe heraus und in die zweite Reinigungsstufe hinein wenigstens einer mechanischen fest/flüssig-Trennoperation unterworfen wird.

Hintergrund der beanspruchten Erfindung ist der überraschende Befund, daß die im Rahmen des erfindungsgemäßen Verfahrens erzeugten Roh-Schmelzen nicht wirklich frei von Feststoffen sind, sondern trotz des Einsatzes von Polymerisationsinhibitoren geringste Mengen an in disperser (teilweise kolloider) Verteilung befindlichem, in unerwünschter Weise gebildetem, visuell praktisch nicht wahrnehmbarem, Polymerisat des wenigstens einen zu reinigenden Monomeren enthalten, das bei über längere Zeit kontinuierlich (bzw. halbkontinuierlich) durchgeführtem Reinigungsverfahren die Blockade bewirkt. Besonders kritisch ist der geschilderte Sachverhalt dadurch, daß diese Polymerisate häufig nicht besonders hochmolekular und deshalb vielfach klebrig sind.

Das erfindungsgemäße Verfahren eignet sich insbesondere dann, wenn der Schmelzpunkt des relevanten Monomeren ≤ 200°C, bevorzugt ≤ 150°C und besonders bevorzugt ≤ 100°C bzw. 5 bis 20°C beträgt. Es ist insbesondere zur Herstellung gereinigter Schmelzen von Acrylsäure, Methacrylsäure, Alkylestern der vorgenannten Säuren und N-Vinylpyrrolidon geeignet.

Als erfindungsgemäß anzuwendende mechanische Trennoperation kommen prinzipiell alle Trennoperationen in Betracht, die zur Abtrennung von Feststoffen (insbesondere von feinteiligen) von Flüssigkeiten geeignet sind. Besonders geeignet sind filtrierende und zentrifugierende Trennoperationen. Als Filtermaterialien können dabei Spaltsiebe, Lochsiebe, Siebgewebe, Filtervliese, Filtergewebe, Faserschichten, Sintermaterialien oder Schüttschichten (z.B. aus Sand) verwendet werden.

Die Porengröße des verwendeten Filtermaterials beträgt für das erfindungsgemäße Verfahren in anwendungstechnisch zweckmäßiger Weise 50 bis 1000 µm. Häufig sollte es 100 bis 500 µm betragen. Vielfach ist aber auch ein Bereich von 10 bis 20 µm oder darunter anzuwenden.

Die Filtration kann als Druck- oder als Vakuumfiltration praktiziert werden. Selbstverständlich kann sie auch als Siebzentrifugieren praktiziert werden. Sedimentierende Einrichtungen (Dekanter, Hydrozyklone, Lamellenklärer, Verweilzeitbehälter) sind für das erfindungsgemäße Verfahren weniger bevorzugt. Das Filter kann erfindungsgemäß auch unmittelbar im Auslaß der ersten Reinigungsstufe angebracht sein.

Das erfindungsgemäße Verfahren eignet sich insbesondere dann, wenn die Roh-Schmelze einer Suspensionskristallisation unterworfen und die resultierende Kristallsuspension wie in der DE-A 10039025 beschrieben mit Hilfe einer Waschkolonne in Restschmelze und Kristalle getrennt wird.

Dies gilt insbesondere dann, wenn das relevante Monomere Acrylsäure ist und auf dem Weg der heterogen katalysierten Gasphasenoxidation wie in der DE-A 19909923 beschrieben hergestellt worden ist.

Beispiele und Vergleichsbeispiele
A) Analog zu Beispiel 2 der DE-A 19909923 wurden durch fraktionierte Kondensation eines abgekühlten Produktgasgemisches einer zweistufigen heterogen katalysierten Gasphasenoxidation von Propen 1 t/h einer Roh-Acrylsäure (Roh-Schmelze) des nachfolgenden Inhalts erzeugt:

| | |
|---|---|
| Acrylsäure | 97,3 Gew.-%, |
| Essigsäure | 0,8 Gew.-%, |
| Propionsäure | 500 Gew.-ppm, |
| Furfural | 700 Gew.-ppm, |
| Maleinsäureanhydrid | 40 Gew.-ppm, |
| Benzaldehyd | 200 Gew.-ppm, |
| Wasser | 1,3 Gew.-%, |
| Phenothiazin (Polymerisationsinhibitor) | 150 Gew.-ppm. |

Die erhaltene Roh-Schmelze war transparent und gemäß visueller Betrachtung frei von Feststoffen. Sie wurde kontinuierlich einem Suspensionskristallisator zugeführt. Dazu wurden Chemienormpumpen vom Typ CPK (das sind Kreiselpumpen mit doppelter Gleitringdichtung), wie sie die Firmen KSB oder Allweiler herstellen, verwendet. Der Suspensionskristallisator war ein Kühlscheibenkristallisator (7 Kühlscheiben, insgesamt ca. 16 m² Kühlfläche, der Durchmesser der kreisrunden Kühlscheiben betrug 1,25 m, 2500 1 Innenvolumen). Die Zulauftemperatur der Roh-Schmelze betrug 25°C. Die Kristallisationswärme wurde über die Kühlflächen abgeführt. Die Restschmelze wurde beim Durchgang durch den Kühlscheibenkristallisator auf 9°C abgekühlt. Aus dem Suspensionskristallisator heraus wurde die Kristallsuspension, die einen Feststoffanteil von etwa 25 % ihres Gewichtes aufwies, kontinuierlich auf eine 2-stufige Schubsiebzentrifuge gegeben (Schubzentrifugen findet man z.B. beschrieben in der Broschüre WB210/11.92 AL der Firma Siebtechnik, D-Mülheim an der Ruhr; bei einer 2-stufigen Schubsiebzentrifuge sind eine rotierende (größere) Außensiebtrommel (repräsentiert die zweite Stufe) und eine (kleinere) rotierende Innensiebtrommel (repräsentiert die erste Stufe) konzentrisch angeordnet; die Außensiebtrommel führt nur Drehbewegungen aber keine Schubbewegung aus; die Innensiebtrommel läuft mit gleicher Drehzahl um wie die Außensiebtrommel und wird zusätzlich durch einen hydraulischen Schubkolben in axialer Richtung hin und her bewegt; beide Trommeln weisen zur Ableitung der Flüssigkeit Siebstruktur auf; in der Aufgabezone der Innensiebtrommel wird sofort der größte Teil der Restschmelze durch die Sieböffnungen abgeschleudert; der Feststoff bleibt als Filterkuchen auf dem Sieb zurück; beim axialen Rückgang der Innentrommel wird am freien Trommelende eine der Schublänge entsprechende Menge Feststoff in die Außensiebtrommel abgeworfen und dort weiter entfeuchtet; beim axialen Vorgehen der Innensiebtrommel wird der Filterkuchen schrittweise in der Außensiebtrommel (die in der Regel länger ist als die Innensiebtrommel) weitergeschoben und schließlich in eine Fangrinne abgeworfen), auf der das Suspensionskristallisat von der Restschmelze abgetrennt wurde. Der Innendurchmesser der ersten Stufe betrug 200 mm. Die Siebspaltweite der ersten Stufe betrug 250 µm. Die zweite Stufe war konisch gestaltet (der Innendurchmesser erweiterte sich von 250 mm auf 310 mm, die Siebspaltweite betrug 300 µm). Die Drehzahl betrug 2200 Umdrehungen je Minute. Die Hubzahl der Innensiebtrommel lag bei 70 pro Minute.
Nach Aufschmelzen der abgeworfenen Kristalle in einem Behälter wurde eine gereinigte Acrylsäureschmelze erhalten, deren Acrylsäuregehalt > 99 Gew.-% betrug.
Nach etwa dreiwöchigem kontinuierlichem Betrieb der Zentrifuge kam es zum Überschiessen der Suspension auf der ersten Stufe. D.h., die Flüssigphase wurde nicht mehr ausreichend auf der ersten Stufe abgetrennt und strömte kanalartig über den auf der ersten Stufe ausgebildeten Filterkuchen in die zweite Stufe. Dies ist insofern von Nachteil, als damit ein Anstieg der Restfeuchte des aus der zweiten Stufe abgeworfenen Kristallisats (von etwa 7 Gew.-% auf > 10 Gew.-%) und ein unruhiger Lauf der Zentrifuge (bedingt durch die Unwucht aufgrund der Kanalbildung und Filterkuchendeformation auf der ersten Stufe) einhergeht, der zu einer Drehzahlreduktion zwingt.
Nach dem Einbau zweier Wechselfilter (Siebkorbfilter aus Edelstahl mit jeweils 550 cm² Siebfläche und 150 µm Siebweite) in den Zulauf von der fraktionierten Kondensation zum Suspensionskristallisator wurde unter ansonsten identischen Betriebsbedingungen auch nach mehr als viermonatiger Betriebsdauer kein Überschiessen der Suspension auf der ersten Stufe festgestellt. Beim Wechsel (der ca. wöchentlich vorgenommen wurde) der Wechselfilter enthielten diese als gummiartiges Polymer Polyacrylsäure. Diese konnte mit wäßriger Natronlauge und nachfolgend reinem Wasser auf dem Wechselfilter ausgewaschen werden.
B) Analog zu Beispiel 1 der DE-A 3641996 wurde als Destillationskopfprodukt einer technischen Acrylsäure eine Acrylsäure (Roh-Schmelze) der Reinheit 99,5 Gew.-% (Gehalt an Acrylsäure) erzeugt, die 200 gew.-ppm an Monomethylether des Hydrochinons als an Polymerisationsinhibitor zugesetzt enthielt und einer 1-Rohr-Fallfilm-Schichtkristallisationsanlage (die Länge des Kristallisatorrohres betrug 6 m, der Innendurchmesser betrug 70 mm, das Rohrmaterial war V2A-Stahl, als externes Temperiermittel wurde ein Gemisch aus 50 Gew.-% Wasser und 50 Gew.-% Ethylenglycol mit einer Temperatur von -20 bis +35°C verwendet; ansonsten wurde wie in der EP-A 616998 verfahren; das Abschmelzen erfolgte bei einer Schichtdicke von 6 bis 12 mm) zum Zweck der kristallisativen Weiterreinigung mit einer Zuführtemperatur von 25°C zugeführt wurde. Die Zuführung erfolgte mit Chemienormpumpen von Typ CPK (das sind Kreiselpumpen mit doppelter Gleitringdichtung), wie sie die Firmen KSB oder Allweiler herstellen. Die so zugeführte Roh-Schmelze war transparent und gemäß visueller Betrachtung frei von Feststoffen. Am oberen Ende wies das Kristallisationsrohr einen Aufgabekranz mit Öffnungen einer Längstausdehnung von ca. 3 mm auf, über die das Kristallisatorrohr mit der Roh-Schmelze als Rieselfilm beschickt wurde. Nach zweiwöchigem Betrieb trat eine partielle Blockade des Aufgabekranzes auf.

Nach Einbau eines Siebkorb-Filters (ca. 100 cm² Filterfläche, 500 µm Siebweite) in den Zulauf zum Kristallisationsrohr traten unter ansonsten identischen Bedingungen innerhalb von 8 Betriebswochen keine Probleme mehr auf. Beim Wechsel des Siebkorb-Filters enthielt dieses als gummiartiges Polymer Polyacrylsäure. Diese konnte mit wäßriger Natronlauge und nachfolgend reinem Wasser aus dem Wechselfilter ausgewaschen werden.

## Patentansprüche

1. Verfahren zur Herstellung einer gereinigten Schmelze wenigstens eines Monomeren, ausgewählt aus der Gruppe umfassend Acrylsäure, Methacrylsäure und N-Vinylpyrrolidon, bei dem man in einer ersten Verfahrensstufe eine das wenigstens eine Monomere enthaltende Gas- oder Flüssigphase erzeugt, in einer ersten Reinigungsstufe das wenigstens eine Monomere aus der Gas- oder Flüssigphase durch Kondensation, Absorption oder Extraktion unter Erhalt einer Roh-Schmelze des wenigstens einen Monomeren abtrennt und anschließend in einer zweiten Reigungsstufe das wenigstens eine Monomere aus der Roh-Schmelze kristallisativ abscheidet, **dadurch gekennzeichnet, daß** die Roh-Schmelze auf ihrem Weg aus der ersten Reinigungsstufe heraus und in die zweite Reinigungsstufe hinein wenigstens einer mechanischen fest/flüssig-Trennoperation unterworfen wird.

2. Verfahren nach Anspruch 1, **dadurch gekennzeichnet, daß** die zweite Reinigungsstufe in einem Fallfilmkristallisator ausgeführt wird.

3. Verfahren nach Anspruch 1, **dadurch gekennzeichnet, daß** die zweite Reinigungsstufe in einem Suspensionskristallisator ausgeführt wird.

## Claims

1. A process for the preparation of a purified melt of at least one monomer selected from the group comprising acrylic acid, methacrylic acid and N-vinylpyrrolidone, in which a gas or liquid phase comprising the one or more monomers is produced in a first process stage, the one or more monomers are separated from the gas or liquid phase by condensation, absorption or extraction to give a crude melt of the one or more monomers in a first purification stage and the one or more monomers are then separated from the crude melt by crystallization in a second purification stage, wherein the crude melt is subjected to at least one mechanical solid/liquid separation operation on its way out of the first purification stage and into the second purification stage.

2. The process according to claim 1, wherein the second purification stage is carried out in a falling-film crystallizer.

3. The process according to claim 1, wherein the second purification stage is carried out in a suspension crystallizer.

## Revendications

1. Procédé de préparation d'une masse fondue purifiée d'au moins un monomère choisi parmi le groupe comprenant de l'acide acrylique, de l'acide méthacrylique et de la N-vinylpyrrolidone, dans lequel on produit dans une première étape une phase gazeuse ou liquide contenant ledit au moins un monomère, on isole dans une première étape de purification ledit au moins un monomère à partir de la phase gazeuse ou liquide par condensation, absorption ou extraction, avec obtention d'une masse fondue brute dudit au moins un monomère et ensuite, dans une deuxième étape de purification, on sépare ledit au moins un monomère à partir de la masse fondue brute par cristallisation, **caractérisé en ce que** la masse fondue brute est soumise à au moins une opération de séparation solide/liquide mécanique sur son trajet depuis la première étape de purification et dans la deuxième étape de purification.

2. Procédé suivant la revendication 1, **caractérisé en ce que** la deuxième étape de purification est effectuée dans un dispositif de cristallisation à film descendant.

3. Procédé suivant la revendication 1, **caractérisé en ce que** la deuxième étape de purification est effectuée dans un dispositif de cristallisation en suspension.
